# EUROPEAN PATENT APPLICATION

(11) **EP 2 898 894 A1**
(43) Date of publication of application: **29.07.2015**
(21) Application number: 14152624.4
(22) Date of filing: 27.01.2014
(51) Int. Cl.: A61K 38/00, A61K 9/50, A61K 9/51

(54) **Nano-in-micro particles for intradermal delivery**

(71) Applicant: LTS LOHMANN Therapie-Systeme AG, 56626 Andernach (DE)
(72) Inventor: Winter, Gerhard, 82377 Penzberg (DE); Engert, Julia, 80993 München (DE); Deng, Yibin, Suzhou 215123, Jiangsu, (CN)
(74) Representative: Simandi, Claus

(57) **Abstract**

The present invention relates to nano-in-micro particles for intradermal delivery of active substances. The nano-in-micro particles according to the invention comprise a matrix of microparticles and nanoparticles carrying at least one active substance and wherein the nanoparticles are dispersed in the matrix of microparticles. In a preferred embodiment of the invention the nano-in-micro particles comprise a densifier.

The invention in particular relates to nano-in-micro particles wherein the nanoparticles carry at least one vaccine and the use of the respective nano-in-micro particles for immunization. The invention further relates to the preparation of such nano-in-micro particles and the use thereof. In addition, the invention relates to compositions comprising these nano-in-micro particles, in particular pharmaceutical compositions.

## Description

The present invention relates to nano-in-micro particles for intradermal delivery of active substances. The nano-in-micro particles according to the invention comprise a matrix of microparticles and nanoparticles carrying at least one active substance and wherein the nanoparticles are dispersed in the matrix of microparticles. In a preferred embodiment of the invention the nano-in-micro particles comprise a densifier.

The invention in particular relates to nano-in-micro particles wherein the nanoparticles carry at least one active agent and the use of the respective nano-in-micro particles is for immunization. The invention further relates to the preparation of such nano-in-micro particles and the use thereof. In addition, the invention relates to compositions comprising these nano-in-micro particles, in particular pharmaceutical compositions.

### Background of the invention

The skin is the most important organ for protection of the human body, and it is also a very important target for drug and vaccine delivery. The stratum corneum is the biological barrier for delivering molecules through the skin. It is necessary to break the stratum corneum in order to deliver macromolecules such as proteins into the skin. Intradermal delivery is one method that can fulfil this task. The administration of active substances by the intradermal route has several advantages compared to the other routes such as intramuscular (i.m.), subcutaneous (s.c.) or intravenous (i.v.) application. First, intradermal delivery, like needle-free intradermal injection and intradermal injection, is a minimally invasive method, avoiding needle injection that may increase the risk of transmission of blood-borne diseases due to the reuse of disposables and improper hygienic conditions. Second, it is a convenient method that can be carried out by non-professionals and even patients themselves, making it specifically suitable for delivery of highly potent drugs (e.g. proteins or nucleotides) and vaccination. Third, it has better pharmacodynamics for treatment of certain skin diseases and in particular for vaccination via the skin.

The skin is an attractive target for vaccine delivery as a dense network of immune-competent cells resides in the viable epidermis. These antigen-presenting cells, so-called Langerhans cells (LCs), are able to engulf pathogens, process them and efficiently present them to naive T-cells and induce effective immune responses [1]. In addition, the epidermis lacks nerve endings and blood vessels which make this site for needle-free administration even more attractive. Targeting of antigens delivered by the intradermal route to skin dendritic cells makes it possible to enhance vaccine efficacy, even better compared to vaccines given by the conventional subcutaneous or intramuscular route [2, 3].

There are several intradermal delivery methods known in the art, mainly including microinjection, microneedles, liquid jet injection, powder particle injection, gold particle injection [4]. Microinjection and liquid jet injection can be applied for liquid formulations. As for vaccination, present vaccines are mostly in liquid formulation, making it also convenient for mass vaccination by this way. The BD SoluviaTM (Becton Dickinson, Franklin Lakes, NJ, USA) is a product for intradermal microinjection and has been used to administer a seasonal influenza vaccine known as Intanza® (Sanofi Aventis, Germany) to elderly patients [5]. Another example is the PharmaJet® device that can be used to inject off-the-shelf licensed vaccines. The biggest disadvantage for liquid vaccines is the requirement of a cold chain for transportation and storage. Furthermore, liquid jet injection is not pain free and depending on individual perception may even be more painful than injection via a very fine needle. Especially for children, where subcutaneous fat tissue is often not yet well developed, jet injection may reach muscular tissue and cause pain or more serious irritation.

Therefore, solid formulation of vaccines and the methods for delivering solids have been developed and are still under continuous development. One potential benefit of solid dry vaccines is the stability at storage temperatures above the usual 2-8°C. Microneedle arrays are very interesting minimally invasive devices for transdermal delivery of drugs and vaccine delivery. There are many investigations going on to test the feasibility using microneedles for vaccination [6-8]. However, more work is needed to formulate antigens in microneedles and improve their mechanical stability. For so-called coated microneedles, where a needle made of steel, or similar carrier is coated with a vaccine matrix, it is not easy to control the load on the needle and the release from the needle when the needle array is poked into the skin and withdrawn. Alternatively, dissolvable needles made of polymers or other biocompatible matrices can be used, where it is intended that the needles break off or dissolve when inserted into the skin and remain there. But such needles are rather difficult to manufacture because of their limited mechanical stability. In addition, protein stability in solid formulations of vaccines is enhanced compared to protein stability in liquid formulations of vaccines.

Epidermal powder immunisation as another method for delivering solid vaccines, including powder particle injection and gold particle injection, and it is a very promising needle-free administration route. The so-called "Gene gun" has been used to deliver DNA-coated gold particles into skin for the purpose of vaccination [9]. The "PowderJect" device has been developed by PowderMed Vaccines Inc. and the Bellhouse group at Oxford University as a multi-use research device for particle-mediated epidermal delivery (PMED) [10]. Gold particles (1-3 µm) are especially suitable for coating DNA antigens and then penetrate into skin by powder injection, while microparticles (20-70 µm) are good candidates to be used for formulating protein and peptide antigens for epidermal powder immunisation [11, 12].

Vaccination technology is one of the most important accomplishments for reducing mortality and improving human health [13]. Since the first vaccination attempt of Jenner in 1796, the administration of vaccine formulations to prevent spreading of infectious diseases has become routine, at least in the developed world. The occurrence of some infectious diseases has been reduced due to widespread vaccination programmes in many countries. To date, most vaccines have been developed successfully by applying live attenuated organisms, inactivated toxins and killed whole organisms. However, these three kinds of vaccine may have some safety issues due to the possibility of the antigen to revert back to active forms [14]. Subunit vaccines such as protein and peptide antigens are alternatives. However, these kinds of vaccines require the help of adjuvants and delivery systems of vaccines [14].

The key role of vaccines to play after immunisation is inducing innate and especially adaptive immune responses, the latter of which includes humoral and cellular immunities [15], resulting in long-term memory B cells or T cells that can quickly activate specific immune response when encountering the same antigens. Vaccines based on particulate delivery systems have been recognised to play a very important role on the activation of protective immunities [18-20]. Particularly, nanoparticles have been widely investigated as delivery platforms and as adjuvants [14]. Nanoparticulate carriers can improve the immunstimulatory action of antigens and adjuvants due to their specific or nonspecific effect on cellular uptake processes, distribution in the body, presentation of the antigens and adjuvants in a specific geometric order, etc.

Particulate delivery systems, such as polymeric particles, inorganic nanoparticles and hybrid particles, have the advantages of controlled and targeting delivery, and acting as vaccine adjuvants [14, 18, 21]. They can stabilize antigens and adjuvants, they can release them in a controlled fashion over time or even according to a trigger (like pH, etc.). Various nanoparticles have been designed and functionalized for delivering antigens/drugs with different purposes. Reddy et al. used pluronic-stabilized polypropylene sulphide (PPS) nanoparticles conjugated to ovalbumin to exploit lymphatic transport and complement activation [19]. Ultra-small PPS nanoparticles (25 nm) have been reported to show stronger immune response than 100 nm PPS nanoparticles because the former can be efficiently taken up by lymphatic vessels and then transported to the draining lymph node [19]. Bourquin et al. studied gelatine nanoparticles for delivering immunostimulatory RNA oligonucleotides in mouse tumor models, successfully triggering an efficient antitumoral immune response [22]. Poly(lactic acid) (PLA) nanoparticles modified with polyvinyl alcohol (PVA), alginate (ALG) and glycolchitosan (GCS) were prepared and investigated as Streptococcus equi antigen carriers [23]. Trimethyl chitosan-hyaluronic acid nanoparticles loaded with ovalbumin showed enhanced immunogenicity after nasal and intradermal vaccination [24]. Biodegradable poly(γ-glutamicacid) nanoparticles loaded with ovalbumin were proven to effectively induce innate and adaptive immunity, suggesting antigen carrier capacity and potential adjuvant function. Some inorganic nanoparticles have also been considered for vaccine/drug delivery. Gold nanoparticles for loading DNA antigens are often used for particle-mediated epidermal delivery [25-27]. Calcium phosphate nanoparticles have been reported as a promising vaccine delivery system and adjuvant [28]. Mesoporous silica nanoparticles (MSNP) are promising systems for enhancing the cytotoxicity of anticancer platinum drug by facilitating the cellular uptake of drug-loaded MSNP [29, 30]. Poly(ethylene imine) (PEI) modified hybrid mesoporous silica nanoparticles have been used for targeting cancer cells [31]. Silica nanoparticles have been extensively used for drug delivery and biomedical applications [32, 33], but not well investigated as a parenteral delivery system.

The development of the technology for the synthesis has greatly improved the controllability of the properties of mesoporous silica nanoparticles with different mesostructures and pore sizes [34]. In fact, silica showed an adjuvant effect in the immunological response of the guinea pig [35]. Vallhov et al. studied the effects of mesoporous silica particles (270 nm and 2.5 µm) on human monocyte-derived dendritic cells (MDDC) and found good cellular uptake of both particles and low-toxicity profiles on MDDC [36]. Recently, Vallhov et al. suggested that mesoporous silica particles have adjuvant properties of tuning effector T cell development according to cell culture experiments [37]. Furthermore, mesoporous silica nanoparticles can enhance mucosal and systemic immune responses when applied by oral immunisations in mouse models [38]. Although silica nanoparticles show potential as a vaccine delivery system, there is no study investigating the effect of them on epidermal powder immunisation.

Poly(lactic-co-glycolic acid)(PLGA)is a kind of biodegradable polymer, which has been approved for use in human by US FDA and European Medicine Agency. PLGA has been extensively investigated as a drug delivery system for macromolecules [39]. The rapid endo-lysosomal escape of PLGA nanoparticles after delivery into cells indicates good implications for gene and drug delivery [40]. PLGA-based particles have shown to be a good candidate as a vaccine delivery system. Cruz et al. found that nano-sized PLGA particles but not microparticles can specifically deliver antigen to human dendritic cells [41]. Wang et al. loaded the foot and mouth disease virus (FMDV) DNA vaccine on chitosan-coated PLGA nanoparticles, which can induce protective immunity again FMDV challenge by intranasal delivery [42]. Sustained release of antigens from PLGA nanoparticles improves the long-term appearance of effector memory cellular response [43].

Gelatin is a biomaterial suitable for drug or vaccine delivery. Gelatin nanoparticles can be well uptaken by murine dendritic cells indicating the potential to be used for vaccine delivery [44]. Cationic gelatine nanoparticles absorbed CpG oligonucleotides can be delivered into the target cells to improve immunostimulatory effects of CpG oligonucleotides both in vitro and in vivo [45]. Furthermore, RNA oligonucleotides loaded onto cationic gelatine nanoparticles were delivered into both myeloid and plasmacytoid dendritic cells, resulting in the production of the antitumoral cytokines IL-12 and IFN-α [22]. Recently, gelatine nanoparticles loaded with CpG oligonucleotides were successfully used in the immunotherapy of allergic horses [46].

Recombinant spider silk particles have been used for delivery of small molecules and proteins showing promising potential as a carrier system [47, 48]. The loading amount of lysozyme as a model protein in a silk protein can be up to 30% with almost 100% loading efficiency [47].. Silk fibroin derived from Bombyx mori cocoons was used to stabilize a live attenuated measles, mumps, and rubella vaccine (MMR® II) by lyophilisation preparation [49], which may save the cost by eliminating the cold chain for transportation. Silk microneedles showed controlled release of a model protein, horseradish peroxidase, indicating the possibility of sustained release of macromolecular antigens from such matrices and providing first evidence, that such systems might well be applicable for intradermal vaccination.

Lipid-based drug delivery systems have already been extensively used to improve the availability of low-soluble drugs and the stability of bioactive macromolecules. Solid lipid nanoparticles (SLN) are a important delivery system of peptides and proteins increasing their stability and therapeutic effect [50]. SLN can be both a vaccine delivery system and a potential new adjuvant for vaccines. SLN carrying Hepatitis B surface antigen induces immune response indicated in vitro and in vivo studies [51].

Considering the intradermal application of one or more of such preferable nanocarriers for vaccination, the man of skill in the art is faced with a serious technical problem. The nanoparticles such as silica, PLGA, gelatine, silk and solid lipid cannot be delivered into epidermal layer of human skin by direct powder injection because smaller particles require higher velocity[52], which is limited by the particle-accelerating device. Small particles like nanoparticles have not enough impact to break the stratum corneum barrier. Kendall et al. proposed a relation between the particle penetration depth and the particle impact parameters (ρνr) that are determined by particle density (p), velocity (v) and radius (r) [52]. As indicated from the penetration of gold particles into human skin, ρνr should be 7-12 kg/m·s for delivering them into the viable epidermal layer [52].

Therefore, it is necessary to either accelerate nanoparticles to a technically not feasible extremely high velocity, or to leave the concept of nanoparticulate carriers and use microparticles, which can be accelerated to a velocity that overall builds up enough impact to break the stratum corneum barrier. A further concept could be to apply extremely high density material, as it has been done with the DNA-coated gold particles. All of these methods have certain disadvantages and limitations. Extreme acceleration devices cannot be built without extremely high costs and lead to unhandy large devices. Using gold carriers leads to rather expensive carriers that can only carry a very small payload. However, for vaccination only a few µg of the respective antigen are necessary. For Using microparticles instead of nanoparticulate carriers, reduce the efficacy of the vaccine.

In view of this serious problem there is need for a novel intradermal delivery technology which is preferably suitable for direct powder injection and allows to deliver active substances into the epidermal layer of the human skin.

The present invention provides novel particles for intradermal delivery of active substances which fulfil this requirement. The particles according to the invention comprise both, microparticles and nanoparticles. They display the advantages of nanoparticles for drug delivery and in addition, they are heavy enough to penetrate the epidermal layer of the human skin.

The invention relates to nano-in-micro particles for intradermal delivery of active substances comprising nanoparticles carrying at least one active substance, characterized in that the nanoparticles are dispersed in a matrix of microparticles.

The present invention relates to nano-in-micro particles for intradermal delivery of active substances comprising a matrix of microparticles and nanoparticles carrying at least one active substance, characterized in that the nanoparticles are dispersed in the matrix of microparticles.

According to the invention, the matrix is a microparticulate carrier. This microparticulate carrier is loaded with nanoparticles. The nanoparticles are subcarriers for the active substance to be delivered. The active substance is loaded into or onto the nanoparticulate subcarrier and/or dispersed in the microparticulate matrix, or loaded in a way that represents a mixture of the mentioned loading patterns.

Such nano-in-micro particles have not been applied to intradermal vaccination before. The nano-in-micro particles provide long-term stability of the nanoparticles and the active substances to be delivered. The active substance is chemically, physically and mechanically stable in the nano-in-micro particles.

The use of nanoparticles as carriers for the active substance has several advantages. Nanoparticulate carriers can for example improve the immunstimulatory action of antigens and adjuvants due to their specific or nonspecific effect on cellular uptake processes. The active substance is presented in a specific geometric order. Furthermore, they have the advantage of controlled and targeted delivery of the active substance. Nanoparticulate carriers can stabilize the active substance. They can release it in a controlled fashion over time or even according to a trigger (like pH, etc.).

The present invention therefore provides a novel technology for intradermal delivery of active substances that displays all the favourable characteristics which are linked to the use of nanoparticulate carriers. However, the inventive concept of nano-in-micro particles allows the delivery of nanoparticles carrying active substances via the skin and therefore opens the way for this administration route to nanoparticles.

The inventive concept is generally applicable to all routes of administration, except of oral administration, and to all kinds of active substances. The size and structure of the nanoparticles can be easily adjusted to the size of the active substance so that the pores can take up the active substance. Also the size and structure of the microparticulate matrix can be easily selected in order to allow the nanoparticulate subcarriers to be released or dispersed from the matrix into skin or other biological compartments, tissues etc..

In a preferred embodiment of the invention the microparticles in the nano-in-micro particles have a size of 1 to 500 micrometer in at least one dimension. In another embodiment the microparticles have a size of 10 to 500 micrometers in two or three dimensions. The microparticles preferably can have a size of 20-70 µm, for example 20, 30, 40, 50, 60, 70, 80, 90 or 100 µm in one, two or three dimensions, preferably 20-40 µm. The microparticles can for example be dense or porous or shell-like, layered, ball shaped or stretched, more preferably they are dense, less porous, and mechanically stable. The surface can be smooth or structured, sharp edged etc.

The size of the microparticles can be determined for example with the methods documented at the National Institute of Standards and Technology (NIST, USA) or the Community Bureau of Reference (CBR). The size of the microparticles can be determined by static light scattering (SLS), light obscuration (LO), scanning electron microscopy (SEM). Pore size can for example be measured by Hg porosimetry.

The microparticles according to the invention can be selected from groups comprising water soluble or not water soluble microparticles, sugar-based microparticles, polysaccharide-based microparticles, dextran-based microparticles or mixtures thereof, microparticles consisting of or comprising a mixture of trehalose, mannitol and dextran (TMD), sucrose microparticles, cyclodextrin microparticles, hydroxyethyl starch (HES) microparticles, amylase microparticles, pectin microparticles, poly(lactic-co-glycolic acid) (PLGA) microparticles, gelatine-hydroxyethyl cellulose microspheres, gelatine microparticles, PLGA-mesoporous silica microspheres, starch microparticles, amylopectin-based starch microparticles, spider silk microparticles, lipid microparticles. In a specific embodiment of the invention the matrix of microparticles comprises sugars or sugar alcohols, with or without a polysaccharide polymer or other polymers.

In a specific embodiment of the invention a matrix of microparticles comprising trehalose, mannitol and dextran (TMD) is used. These microparticles may have different weight ratios of trehalose, mannitol and dextran, for example trehalose, mannitol and dextran in a weight ration of 3:3:4.

In a preferred embodiment of the invention the nanoparticles in the nano-in-micro particles have a size of 1 to 1000 nanometers in at least one dimension. In another embodiment the nanoparticles have a size of 1 to 1000 nanometers in two or three dimensions. The nanoparticles can have a size of 1, 5, 10, 15, 20, 25, 50, 75, 100, 130, 150, 200, 250, 270, 300, 350, 400, 450, 500, 550, 600, 700, 800, 900, 1000, nanometers in one, two or three dimensions.

Nanoparticles can be porous or non-porous. In one embodiment of the invention the nanoparticles can have pore sizes smaller than 2 nm. In another embodiment of the invention nanoparticles can have pore sizes larger than 2 nm.

The size of the nanoparticles can be determined for example with the methods documented at the National Institute of Standards and Technology (NIST, USA) or the Community Bureau of Reference (CBR). Nanoparticle size can be determined by dynamic light scattering (DLS), nanoparticle tracking analysis (NTA), Scanning electron microscopy (SEM). Pore size of the nanoparticles can be determined by SEM, transmission electron microscopy (TEM) and BET.

The nanoparticles according to the invention can be selected, without restriction, for example from the group comprising polymeric nanoparticles, inorganic nanoparticles, hybrid nanoparticles, biodegradable nanoparticles, silica nanoparticles, biodegradable polymers like poly(lactic-co-glycolic acid) (PLGA) nanoparticles, gelatine nanoparticles, silk nanoparticles, solid lipid nanoparticles, pluronic-stabilized polypropylene sulphide (PPS) nanoparticles, ultra-small nanoparticles, poly(lactic acid) (PLA) nanoparticles optionally modified with polyvinyl alcohol (PVA), alginate (ALG) and / or glycolchitosan (GCS), trimethyl chitosan-hyaluronic acid nanoparticles, biodegradable poly(γ-glutamicacid) nanoparticles, gold nanoparticles, calcium phosphate nanoparticles, mesoporous silica nanoparticles (MSNP), mesoporous silica nanoparticles with different mesostructure and pore size, poly(ethylene imine) (PEI) modified hybrid mesoporous silica nanoparticles, biodegradable polymers like poly(lactic-co-glycolic acid) (PLGA) nanoparticles, optionally coated with chitosan, cationic gelatine nanoparticles, recombinant spider silk particles, silk fibroin derived from Bombyx mori cocoons-based nanoparticles, lipid-based nanoparticles, solid lipid nanoparticles (SLN).

In a preferred embodiment of the invention mesophorous silica nanoparticles (MSNP or MSNP-NH2) are used as carriers for active substances. MSNPs with different size in one, two or three dimensions may be used, for example MSNPs with a size of 120 to 140 nm, preferably 130 nm in one, two or three dimensions. In addition, MSNPs with different microstructure and pore size may be used, for example having a pore size of at least 2 nm, preferably around 2.3 nm, 2.5 nm, 5 nm, 10 nm or more.

In another preferred embodiment of the invention PLGA nanoparticles are used as carriers for active substances. PLGA nanoparticles may have different size in one, two or three dimensions, for example 150-200 nm in one, two or three dimensions.

In another preferred embodiment of the invention gelatine nanoparticles are used as carriers for active substances. Such gelatine nanoparticles may have a cationic surface.

In a preferred embodiment of the invention the nano-in-micro particles comprise a densifier.

Typical excipients used for preparation of pharmaceutically applicable nanoparticles and microparticles have a medium to low specific density, of typically around 1.4 g/ml true density, and of course a much lower tap density. In order to adjust the optimal density of the nano-in-micro particles for intradermal delivery densifiers may be added. The densifiers may be added to the microparticles and / or the nanoparticles.

The densifier can elevate the true density of the microparticle/nanoparticle system above 1,4 g/ml, preferably above 1,5 g/ml, more preferably even higher and accordingly the tap density can be elevated above 0,7 g/ml, preferably above 0,8 g/ml, more preferably even higher.

According to the invention, micro- and/or nanoparticulate densifiers can be included into the nano-in-micro particles for intradermal delivery. Such densifiers need to fulfil several properties, like biocompatibility, dense structure, inertness against the nano- and microcarriers used and the active substances to be delivered like for example against vaccines and / or adjuvants. According to the invention, dense salts, like, calcium carbonate, magnesium carbonate, barium sulphate, hydroxylapatite and its derivatives and similar representatives of inert, dense, inorganic salts with low aqueous solubility fulfil the necessary conditions. Therefore, in one embodiment of the invention the nano-in-micro particles comprise at least one densifier selected from the group comprising microparticulate densifiers, nanoparticulate densifiers, gold particles, dense salts like Calium carbonate, Magnesium carbonate, Barium sulphate, hydroxylapatite and its derivatives, magnetite, metal oxides, inert metals, ceramics, hybrid anorganic materials.

Different amounts of densifier can be used, for example 5-90 %, 10-80%, 15- 50%. I a preferred embodiment 10-15 wt.-% of densifier are used. The densifier can be added to the microparticulate matrix or the densifier can be added to the nanoparticulate carrier or to both. In a preferred embodiment of the invention CaCO₃ is used as densifier, for example CaCO₃ is added to the TMD matrix.

In one embodiment of the invention the active substance to be delivered by the nano-in-micro particles is selected from the group comprising chemical compounds, natural products, extracts of natural products, proteins, peptides, antibodies, insulin, vaccines, subunit vaccines, protein antigens, peptide antigens, live attenuated organisms, inactivated toxins, killed whole organisms, vaccines against influenza, vaccines against cancer, vaccines against viruses, vaccines against human immunodeficiency virus (HIV), vaccines against Hepatitis A, B or C, nucleic acids, DNA, RNA, siRNA, antisense oligonucleotides, steroids, steroid derivatives, adjuvants.

The nanoparticulate carrier can carry or may comprise more than only one active substance, for example at least two different active substances. The invention relates to nano-in-micro particles carrying 1, 2, 3, 4, 5 or more different active substances.

In a preferred embodiment at least one active substance in the nano-in-micro particles is a pharmaceutical compound (a drug). The pharmaceutical compound is for example selected from the group comprising proteins or peptides like antibodies, in a preferred embodiment at least one active substance is a vaccine.

In another embodiment, the nano-in-micro particles comprise an adjuvant, for example they comprise a vaccine or an antibody or an antibody derivative as at least one active substance and an adjuvant. However, the nanoparticulate carrier may itself be the adjuvant or have an adjuvant effect.

In one embodiment of the invention silica nanoparticles are used. The pores of silica nanoparticles provide a controlled space for loading active substance. The size of the pores can be adjusted to the size of the active substance. In a preferred embodiment of the invention silica nanoparticles carry adjuvants, peptide antigens and / or protein antigens if silica particles with large pores are used.

In another aspect, the present invention relates to a composition for intradermal delivery comprising nano-in-micro particles according to the invention. For example, nano-in-micro particles comprised in a composition for the intradermal delivery of cosmetics or life style products.

In this connection the present invention further relates to a method for application of such composition, for example the applications of cosmetics or life style products, comprising the steps
a) providing nano-in-micro particles carrying at least one active substance,
b) optionally formulating a composition of nano-in-microparticles in one or more excipients of any kind,
c) intradermal delivery of the composition.

In another aspect, the present invention relates to a pharmaceutical composition for intradermal delivery comprising nano-in-micro particles according to the invention. Pharmaceutical compositions according to the invention can for example be used for the therapeutic application when liquid injection is not indicated for example since the respective patient has not sufficient subcutaneous fat tissue, like in elder persons or children. In addition, a cold-chain requirement for dried vaccines can be circumvented, especially when vaccines have low stability in liquid form or when they have to be stored at higher temperature. Pharmaceutical compositions according to the invention can furthermore be used for the therapeutic application in infectious diseases, cancer or for immunization, for treatment of skin diseases like e.g. psoriasis, atopic dermatitis, lupus or other rheumatoid skin diseases.

A preferred embodiment the invention relates to a pharmaceutical composition comprising nano-in-micro particles according to the invention, a densifier and wherein the nanoparticulate carrier carries a vaccine, an antibody or an antibody derivative and optionally such nano-in micro particles may comprise an adjuvant.

In a preferred embodiment of the invention the nano-in-micro particles are suitable for specific therapeutic application via intradermal delivery, for example for intradermal vaccination and / or mucosal vaccination.

The composition, in particular the pharmaceutical composition according to the invention can comprise further aids, like additives and auxiliaries. The pharmaceutical composition according to the invention can for example comprise one or more adjuvants.

In another aspect the present invention relates to the use of nano-in-micro particles in methods for intradermal delivery of active substances, for example for epidermal power injection, epidermal power delivery or particle-mediated epidermal delivery (PMED) or mucosal powder injection, mucosal powder delivery. In a preferred embodiment mesoporous nanoparticles (MSNPs) are used for immunization by epidermal powder injection. Mesoporous silica nanoparticles have never been used as a vaccine delivery system for epidermal powder immunisation before. Epidermal powder delivery can be used to deliver active substances into superficial layer of skin for the purpose of cosmetics, life style products, skin disease treatment, mucosal administration, novel drug administration routes and most preferably for vaccination.

Therefore, in another aspect the present invention relates to the use of nano-in micro particles according to one of the previous claims for intradermal delivery of cosmetics, life style products, for skin disease treatment, mucosal administration, for drug administration or vaccination.

Microparticulate carriers loaded with nanoparticulate vaccine loaded subcarriers provide a preferred solution of the problem recognized by the inventors. Such nano-in-micro particles have not been applied to intradermal vaccination before. According to the invention, micro- or nanoparticulate densifiers are included into the nano-in-micro carrier system for intradermal delivery, if necessary in order to adjust the density that is required for epidermal powder immunization. The addition of a biocompatible densifier into the nano-in-micro particles increase the density of the nano-in-micro particles, which is good for penetration when injected. Such densifiers need to fulfil several properties, like biocompatibility, dense structure, inertness against the nano- and microcarriers used and the vaccines and adjuvants applied. According to the invention, dense salts, like, calcium carbonate, magnesium carbonate, barium sulphate, hydroxylapatite and its derivatives and similar representatives of inert, dense, inorganic salts with low aqueous solubility fulfil the necessary conditions. The density of the nano-in-micro particles and the stability of encapsulated vaccine are very important factors when preparing microparticles for epidermal powder injection. The objective of the invention is to obtain dense nano-in-micro particles containing antigen-loaded nanoparticles. The nano-in-micro particles according to the invention can be used by the needle-free injection device, for example as described in the EP1599242 B1 [67] or other devices serving the same or similar purpose. Preferably, the nano-in-micro particles should meet the requirements for epidermal or mucosal powder immunisation.

The methods for epidermal powder immunisation include e.g. without limitation intracellular particle delivery delivering small particles such as 1-3 µm gold particles and extracellular particle delivery delivering micro-scale particles (20-70 µm) [53, 54].

The present invention further relates to methods for producing the nano-in-micro particles. Sugar-based microparticles prepared by spray-freeze-drying (SFD) were used for epidermal powder immunisation against influenza, resulting in humoral immune responses in humans [55]. The formulations of sugar-based microparticles were optimized and improved for better particle morphology and higher density that are required for powder injection [12, 56, 57]. Schiffter et al. developed insulin encapsulated microparticles consisting of trehalose, mannitol and dextran for needle-free ballistic powder injection [58]. It is obvious, that such microparticles cannot directly result in intracellular delivery and need to dissolve or disintegrate before their payload can come into action.

There are many possibilities to prepare microparticles that may be useful as delivery systems for powder injection. Microparticles can be prepared by spray-drying (SD), spray-freeze drying (SFD), prilling, spray congealing, droplet formation and precipitation, coacervation, solvent extraction, spraying into a non-solvent, etc.. For example, PLGA microparticles [39] and gelatine-hydroxyethyl cellulose microspheres [59]. PLGA-mesoporous silica microspheres were prepared for DNA prime-protein boost vaccination [60]. However, nanoparticles encapsulated in the biocompatible sugar-based microparticles have not yet been investigated. Therefore, preparation of microparticles with antigens encapsulated in nanocarriers is a promising new approach for epidermal powder immunisation.

The preparation of microparticles encapsulating active substances and their application has been described in the patent literature before, but not comprising the combination of a microparticulate matrix, a nanoparticulate carrier dispersed in that matrix and optionally a densifier added to the microparticulate matrices to allow for optimal ballistic delivery into the skin.

The matrix for the microparticles can be either readily water soluble or not. In the case of a water soluble matrix, the included nanoparticulate carrier is immediately released after exposure to tissue and dissolution of the matrix and can act in its intended way as a vaccine, interact with cells etc. In case of a water insoluble matrix, that degrades and/or dissolves slowly after administration into the body, the vaccine and adjuvant components are released slowly over time and their biological action will be delayed and prolonged.

This can be favourable in certain cases, whereas in other cases, immediate release is preferred. The nano-in-micro particles according to the invention are applicable to various situations one can encounter in different vaccination and delivery situations.

The addition of a biocompatible densifier (e.g. a salt) facilitates penetration during injection. The nano-in-micro particles comprising a water-soluble matrix and nanoparticles loaded with an active substance can be dissolved fast when injected into skin, and then the nanoparticles loaded with active substance, e.g. antigen-loaded nanoparticles are released. The microparticles, when consisting of slowly water soluble or water insoluble but biodegradable materials would dissolve slowly and release the nanoparticles loaded with active substance, e.g. antigen slowly.

The antigen-loaded nanoparticles can increase the internalisation by antigen-presenting cells such as Langerhans cells, dendritic cells and macrophage cells. Antigen-loaded nanoparticles can achieve sustained release of antigens and adjuvants after delivery into skin. The stability of antigens is improved by encapsulated in nanoparticles shielding the stress from spray-freeze-drying or other manufacturing steps towards microparticle production and powder injection steps. The novel nano-in-micro particles combine the advantages of nanoparticles as a vaccine delivery and microparticles used for epidermal powder injection.

In a specific embodiment of the invention, the densifier can also act as a carrier for the vaccine and/or the adjuvant. In this case it is not mandatory, that the vaccine or adjuvant is present on or in a nanoparticle, but can be dispersed in the matrix and attached or adsorbed or mixed with the densifier. Such nano-in-micro particles are specifically simple in composition, they may comprise only very few excipients and are nevertheless advantageous. In an even more preferred embodiment of the nano-in-micro particles, the densifier is present in nanoparticulate size and dispersed in a water soluble microparticulate matrix. In this case, the loading of the vaccine and/or the adjuvant to the nano-in-micro particles can be carried out in different modes. One mode allows to dissolve the vaccine and/or the adjuvant in the aqueous solution of the microparticulate matrix former, adding the densifier, allow the vaccine and/or the adjuvant to adsorb to the densifier and then to produce nano-in-micro particles from the mixture. In another embodiment, the vaccine and/or the adjuvant are first loaded to the densifier and such loaded subcarrier is then admixed to the microparticle matrix former.

All permutations about the respective distribution of the densifier and/or the active substance, e.g. a drug in nanoparticles and/or microparticles are possible and present a specific embodiment of the invention. The active substance can for example be located in the nanoparticles and / or located on the nanoparticles. In addition, the active substance can be located in or on the matrix of microparticles. Furthermore, if a densifier is comprised in the micro-in-nano particles, the densifier can be located either in the nanoparticles, on the nanoparticles or in the matrix of microparticles or on the matrix of microparticles or in a combination thereof.

The following non limiting examples illustrate the possible permutations. However, it is obvious that other combinations are possible.

These combinations of the respective active substance in the nano-in-micro particles are possible:
1) active substance in the nanoparticles (NP)
2) active substance on the NP
3) active substance in and on the NP
4) active substance in the NP and in the matrix of microparticles (MP)
5) active substance on the NP and in the MP
6) active substance in the NP and on the MP
7) active substance on the NP and on the MP
8) active substance in the NP and on the NP and in the MP
9) active substance in the NP and on the NP and on the MP
10) active substance in the NP and on the NP and in the MP and on the (MP)
11) active substance in the NP and in the MP and on the MP
12) active substance on the NP and in the MP and on the MP
13) active substance in the MP
14) active substance on the MP
15) active substance in the MP and on the MP

These combinations of the respective active substance in the nano-in-micro particles which comprise a densifier are possible:
1) active substance in the NP and densifier in the NP and / or on the NP and / or in the MP and / or on the MP
2) active substance on the NP and densifier in the NP and / or on the NP and / or in the MP and / or on the MP
3) active substance in and on the NP and densifier in the NP and / or on the NP and / or in the MP and / or on the MP
4) active substance in the NP and in the matrix of microparticles (MP) and densifier in the NP and / or on the NP and / or in the MP and / or on the MP
5) active substance on the NP and in the (MP) and densifier in the NP and / or on the NP and / or in the MP and / or on the MP
6) active substance in the NP and on the (MP) and densifier in the NP and / or on the NP and / or in the MP and / or on the MP
7) active substance on the NP and on the (MP) and densifier in the NP and / or on the NP and / or in the MP and / or on the MP
8) active substance in the NP and on the NP and in the matrix of microparticles (MP) and densifier in the NP and / or on the NP and / or in the MP and / or on the MP
9) active substance in the NP and on the NP and on the (MP) and densifier in the NP and / or on the NP and / or in the MP and / or on the MP
10) active substance in the NP and on the NP and in the MP and on the (MP) and densifier in the NP and / or on the NP and / or in the MP and / or on the MP
11) active substance in the NP and in the MP and on the (MP) and densifier in the NP and / or on the NP and / or in the MP and / or on the MP
12) active substance on the NP and in the MP and on the (MP) and densifier in the NP and / or on the NP and / or in the MP and / or on the MP
13) active substance in the MP and densifier in the NP and / or on the NP and / or in the MP and / or on the MP
14) active substance on the (MP) and densifier in the NP and / or on the NP and / or in the MP and / or on the MP
15) active substance in the MP and on the (MP) and densifier in the NP and / or on the NP and / or in the MP and / or on the MP

Nano-in-micro particles comprising vaccine (v) and adjuvant (a) and wherein the vaccine might be either on the NP, in the MP or on the NP and in the MP might have the following distribution:
1) v on NP and a in NP
2) v on NP and a on NP
3) v on NP and a in and on NP
4) v on NP and a in MP
5) v on NP and a on MP
6) v on NP and a in and on MP
7) v on NP and a in NP and in MP
8) v on NP and a in NP and on MP
9) v on NP and a in NP and in MP and on MP
10) v on NP and a on NP and in MP
11) v on NP and a on NP and on MP
12) v on NP and a on NP and in MP and on MP
13) v on NP and a in NP and on NP and in Mp
14) v on NP and a in NP and on NP and in MP and on MP
15) v in MP and a in NP
16) v in MP and a on NP
17) v in MP and a in and on NP
18) v in MP and a in MP
19) v in MP and a on MP
20) v in MP and a in and on MP
21) v in MP and a in NP and in MP
22) v in MP and a in NP and on MP
23) v in MP and a in NP and in MP and on MP
24) v in MP and a on NP and in MP
25) v in MP and a on NP and on MP
26) v in MP and a on NP and in MP and on MP
27) v in MP and a in NP and on NP and in Mp
28) v in MP and a in NP and on NP and in MP and on MP
29) v in MP and on NP and a in NP
30) v in MP and on NP and a on NP
31) v in MP and on NP and a in and on NP
32) v in MP and on NP and a in MP
33) v in MP and on NP and a on MP
34) v in MP and on NP and a in and on MP
35) v in MP and on NP and a in NP and in MP
36) v in MP and on NP and a in NP and on MP
37) v in MP and on NP and a in NP and in MP and on MP
38) v in MP and on NP and a on NP and in MP
39) v in MP and on NP and a on NP and on MP
40) v in MP and on NP and a on NP and in MP and on MP
41) v in MP and on NP and a in NP and on NP and in Mp
42) v in MP and on NP and a in NP and on NP and in MP and on MP

Nano-in-micro particles comprising vaccine (v) and adjuvant (a) and wherein the NP is the adjuvant might have the following distribution:
1) v on NP
2) v on NP and in MP
3) v on NP and on MP
4) v on NP and in MP and on MP
5) v in NP
6) v in NP and in MP
7) v in NP and on MP
8) v in NP and in MP and on MP
9) v in NP and on NP
10) v in NP and on NP and in MP
11) v in NP and on NP and on MP
12) v in NP and on NP and in MP and on MP
13) v in MP
14) v on MP
15) v in and on MP

The advantageous embodiments of the invention are explained further within the examples. Sugars and polysaccharides can be chosen as the matrix for preparing microparticles due to good solubility and biocompatibility. Trehalose and mannitol are sugars and dextran with different molecular weights that can be used. Other sugars such as sucrose, cyclodextrin, and polysaccharides such as hydroxyethyl starch (HES), amylase, pectins, anorganic excipients, lipids, polymers of any kind may also preferably be considered in the nano-in-micro particles.

Some examples for further illustrating the invention are described in detail as follows.

### Examples:

### Example 1

In this example, TMD/MSNP-NH2/OVA microparticles were prepared. The weight ratio of trehalose, mannitol and dextran (TMD) in the formulation is 3:3:4. Dextran is used in two qualities, one using a low molecular weight (about 10 kDa), the other using a high molecular weight ( about 100 kDa). The solid content of the trehalose, mannitol and dextran aqueous solution is 35% by mass. The matrix solution can be sterilised by membrane filtration (0.2 µm).

Mesoporous silica nanoparticles were fabricated to load a model antigen, ovalbumin (OVA). Ovalbumin was physically absorbed onto cationized, mesoporous silica nanoparticles (MSNP-NH2) with a size of about 130 nm and a pore size around 2.5 nm. The ovalbumin-loaded silica nanoparticles were added into the TMD matrix former to prepare TMD microparticles, that contain silica nanoparticles and ovalbumin by spray-freeze-drying. For spray freeze drying the matrix solution (see above) together with nanoparticles and ovalbumin was sprayed by means of an ultrasonic nozzle into liquid nitrogen and freeze dried afterwards. The SEM micrographs of pure TMD microparticles and TMD microparticles containing silica nanoparticles and ovalbumin are not shown. The mass ratio of MSNP-NH2 and OVA is 1/2, and the OVA content inside the microparticles is 25 µg per gram of TMD microparticles. Most of the microparticles have sizes around 20-40 µm.

The distribution of added protein was determined by observing fluorescent ovalbumin in the TMD microparticles using CLSM (confocal laser scanning microscopy). Rhodamine B isothiocyanate-labelled ovalbumin (RITC-OVA) and OVA were mixed at a mass ratio of 1/3 and then were added in the formulation, keeping the protein content at 25 µg/mg. The CLSM micrographs showed the homogeneously distributed RITC-OVA in red. The 3D view of Z-stacking micrographs demonstrated the shrinking morphology on surface.

### Example 2

In this example, Influenza H1N1 hemagglutinin (HA) was used as the biologically active substance in the microparticle matrix. First, silica nanoparticles (MSNP-NH2) were added to an aqueous solution of H1N1 HA to allow adsorption of the antigen to the nanoparticles. Then this suspension was combined with TMD, resulting in a suspension containing all ingredients. The suspension was then spray-freeze-dried. The obtained microparticles are flowable similar to the case of ovalbumin. The stability of H1N1 HA was investigated by SDS-PAGE and Western Blot. H1N1 HA from the TMD formulation was prepared by dissolving 10 mg of microparticles in 0.2 ml of PBS buffer (10 mM, pH=7.0). In the Western Blot, anti-influenza H1N1 HA monoclonal antibodies (MAb) was used as the primary antibody, and IRDye 800CW conjugated goat anti-Mouse IgG was the secondary antibody.

Biological activity of H1N1 HA in the TMD microparticle formulation was tested by hemagglutinin inhibition (HAI) assay. Fresh red blood cells (RBCs) were diluted to 0.5% (v/v) and then used in the assay The results show that the biological activity of the H1N1 HA in TMD microparticles is fully maintained.

### Example 3

PLGA nanoparticles loaded with ovalbumin have been prepared. About 10 wt% of ovalbumin was encapsulated by an emulsification method. The PLGA-OVA nanoparticles have a size of 150-200 nm and release OVA in PBS buffer over time. These PLGA nanoparticles were encapsulated in TMD microparticles by spray-freeze-drying. In order to visualize PLGA nanoparticles in the microparticles, Rhodamine B isothiocyanate (RITC) was encapsulated in PLGA nanoparticles (about 200 nm) to fluorescently label them. The distribution of PLGA nanoparticles in the TMD microparticles is very good.

### Example 3 a)

The experiment as described in Example 3 was repeated with PLGA nanoparticles that had been cationized by adding a chitosan coating.

### Example 4

Plain gelatine nanoparticles with a negatively charged surface and cationic gelatine nanoparticles were prepared. Gelatine nanoparticles were encapsulated in TMD microparticles using spray-freeze-drying. The microparticles containing gelatine nanoparticles were investigated by CLSM microscopy.

### Example 5

Trehalose, mannitol, dextran with a low molecular weight of around 10 kDa and dextran with a high molecular weight were used to prepare TMD microparticles. Dextran with molecular weights of 150 kDa and 100 kDa were used as the high molecular weight dextran in the formulation. Different amounts of CaCO₃ (true density ca. 2.7 g/ml) were added in the formulation as shown in Table 1. In the formulation of TMD (100kDa), the tap density was increased from 670 to 831 kg/m³ when the weight ration of TMD and CaCO₃ comes to 2.5/1. In the case of TMD (150kDa), the tap density was increased from 618 to 770 kg/m³ when the weight ration of TMD and CaCO₃ is 5/1. When TMDD and CaCO₃ are used at a weight ratio of 1/1, the density of TMD (100kDa) and TMD (150kDa) increase to 850 kg/m³.

**Table 1 Tap density of sugar-based microparticles**

| Sample | Dextran (100 kDa) | Dextran (150 kDa) |
|---|---|---|
| | Tap density (kg/m3) | Tap density (kg/m3) |
| TMDD (35%(w/w)) | 670 | 618 |
| TMDD/CaCO3=2.5/1 | 831 | 770 |
| TMDD/CaCO3=1/1 | 853 | 848 |

### Example 6

The green fluorescent Alexa Fluor® 488 ovalbumin conjugate (Alexa488-OVA) and ovalbumin were mixed together at a mass ratio of 1/50. The obtained OVA/Alexa488-OVA mixture was added in the trehalose, mannitol, dextran formulation to prepare TMD microparticles containing OVA/Alexa488-OVA. In the formulation containing CaCO₃, CaCO₃ was added at an amount of 10 wt% of the TMD matrix. TMD microparticles containing CaCO₃ and OVA/Alexa488-OVA were obtained by spray-freeze-drying. Both of the microparticles were characterised by CLSM. The distributions of OVA in the TMD microparticles and CaCO₃-enhanced TMD microparticles are homogeneous as indicated from the green spheres. Most of the microparticles of both formulations have sizes around 20-40 µm.

### References

[1] S. Bubiuk, M. Baca-Estrada, L.A. Babiuk, C. Ewen, M. Foldvari, Cutaneous vaccination: the skin as an immunologically active tissue and the challenge of antigen delivery (vol 66, pg 199, 2000), J. Control. Release, 67 (2000) 415-415.
[2] N. Romani, M. Thurnher, J. Idoyaga, R.M. Steinman, V. Flacher, Targeting of antigens to skin dendritic cells: possibilities to enhance vaccine efficacy, Immunol Cell Biol, 88 (2010) 424-430.
[3] F. Sparber, C.H. Tripp, M. Hermann, N. Romani, P. Stoitzner, Langerhans cells and dermal dendritic cells capture protein antigens in the skin: Possible targets for vaccination through the skin, Immunobiology, 215 (2010) 770-779.
[4] E.E. Kis, G. Winter, J. Myschik, Devices for intradermal vaccination, Vaccine, 30 (2012) 523-538.
[5] S.T. Duggan, G.L. Plosker, Intanza 15 mug intradermal seasonal influenza vaccine in older adults (aged >/= 60 years): profile report, BioDrugs, 24 (2010) 407-409.
[6] S.M. Bal, Z. Ding, G.F. Kersten, W. Jiskoot, J.A. Bouwstra, Microneedle-based transcutaneous immunisation in mice with N-trimethyl chitosan adjuvanted diphtheria toxoid formulations, Pharm Res, 27 (2010) 1837-1847.
[7] Z. Ding, E. Van Riet, S. Romeijn, G.F. Kersten, W. Jiskoot, J.A. Bouwstra, Immune modulation by adjuvants combined with diphtheria toxoid administered topically in BALB/c mice after microneedle array pretreatment, Pharm Res, 26 (2009) 1635-1643.
[8] Z. Ding, F.J. Verbaan, M. Bivas-Benita, L. Bungener, A. Huckriede, D.J. van den Berg, G. Kersten, J.A. Bouwstra, Microneedle arrays for the transcutaneous immunization of diphtheria and influenza in BALB/c mice, J Control Release, 136 (2009) 71-78.
[9] L. Frelin, M. Alheim, A. Chen, J. Soderholm, B. Rozell, C. Barnfield, P. Liljestrom, M. Sallberg, Low dose and gene gun immunization with a hepatitis C virus nonstructural (NS) 3 DNA-based vaccine containing NS4A inhibit NS3/4A-expressing tumors in vivo, Gene Ther, 10 (2003) 686-699.
[10] D.H. Fuller, P. Loudon, C. Schmaljohn, Preclinical and clinical progress of particle-mediated DNA vaccines for infectious diseases, Methods, 40 (2006) 86-97.
[11] D. Chen, R.L. Endres, C.A. Erickson, K.F. Weis, M.W. McGregor, Y. Kawaoka, L.G. Payne, Epidermal immunization by a needle-free powder delivery technology: immunogenicity of influenza vaccine and protection in mice, Nat Med, 6 (2000) 1187-1190.
[12] Y.F. Maa, M. Ameri, C. Shu, L.G. Payne, D.X. Chen, Influenza vaccine powder formulation development: Spray-freeze-drying and stability evaluation, J. Pharm. Sci., 93 (2004) 1912-1923.
[13] S.L. Plotkin, S.A. Plotkin, A short histofry of vaccination, in: S.A. Plotkin, W.A. Orenstein (Eds.) Vaccines, WB Saunders Company, Philadelphia, 2004, pp. 1-15.
[14] L.J. Peek, C.R. Middaugh, C. Berkland, Nanotechnology in vaccine delivery, Advanced Drug Delivery Reviews, 60 (2008) 915-928.
[15] K.P. Murphy, P. Travers, M. Walport, C. Janeway, Janeway's immunobiology, Garland Schience, 2008.
[16] B. Pulendran, R. Ahmed, Immunological mechanisms of vaccination, Nat Immunol, 12 (2011) 509-517.
[17] S.P. Kasturi, 1. Skountzou, R.A. Albrecht, D. Koutsonanos, T. Hua, H.I. Nakaya, R. Ravindran, S. Stewart, M. Alam, M. Kwissa, F. Villinger, N. Murthy, J. Steel, J. Jacob, R.J. Hogan, A. Garcia-Sastre, R. Compans, B. Pulendran, Programming the magnitude and persistence of antibody responses with innate immunity, Nature, 470 (2011) 543-U136.
[18] Y.J. Kwon, E. James, N. Shastri, J.M.J. Frechet, In vivo targeting of dendritic cells for activation of cellular immunity using vaccine carriers based on pH-responsive microparticles, P Natl Acad Sci USA, 102 (2005) 18264-18268.
[19] S.T. Reddy, A.J. van der Vlies, E. Simeoni, V. Angeli, G.J. Randolph, C.P. O'Neill, L.K. Lee, M.A. Swartz, J.A. Hubbell, Exploiting lymphatic transport and complement activation in nanoparticle vaccines, Nat Biotechnol, 25 (2007) 1159-1164.
[20] T. Uto, T. Akagi, K. Yoshinaga, M. Toyama, M. Akashi, M. Baba, The induction of innate and adaptive immunity by biodegradable poly(gamma-glutamic acid) nanoparticles via a TLR4 and MyD88 signaling pathway, Biomaterials, 32 (2011) 5206-5212.
[21] C. Vauthier, K. Bouchemal, Methods for the preparation and manufacture of polymeric nanoparticles, Pharm Res, 26 (2009) 1025-1058.
[22] C. Bourquin, C. Wurzenberger, S. Heidegger, S. Fuchs, D. Anz, S. Weigel, N. Sandholzer, G. Winter, C. Coester, S. Endres, Delivery of Immunostimulatory RNA Oligonucleotides by Gelatin Nanoparticles Triggers an Efficient Antitumoral Response, J Immunother, 33 (2010) 935-944.
[23] H.F. Florindo, S. Pandit, L.M. Goncalves, H.O. Alpar, A.J. Almeida, Surface modified polymeric nanoparticles for immunisation against equine strangles, Int J Pharm, 390 (2010) 25-31.
[24] R.J. Verheul, B. Slutter, S.M. Bal, J.A. Bouwstra, W. Jiskoot, W.E. Hennink, Covalently stabilized trimethyl chitosan-hyaluronic acid nanoparticles for nasal and intradermal vaccination, J. Control. Release, 156 (2011) 46-52.
[25] B. Wang, H. Yu, F.R. Yang, M. Huang, J.H. Ma, G.Z. Tong, Protective efficacy of a broadly cross-reactive swine influenza DNA vaccine encoding M2e, cytotoxic T lymphocyte epitope and consensus H3 hemagglutinin, Virol J, 9 (2012) 127.
[26] E.F. Boudreau, M. Josleyn, D. Ullman, D. Fisher, L. Dalrymple, K. Sellers-Myers, P. Loudon, J. Rusnak, R. Rivard, C. Schmaljohn, J.W. Hooper, A Phase 1 clinical trial of Hantaan virus and Puumala virus M-segment DNA vaccines for hemorrhagic fever with renal syndrome, Vaccine, 30 (2012) 1951-1958.
[27] L.C. Dupuy, M.J. Richards, D.S. Reed, C.S. Schmaljohn, Immunogenicity and protective efficacy of a DNA vaccine against Venezuelan equine encephalitis virus aerosol challenge in nonhuman primates, Vaccine, 28 (2010) 7345-7350.
[28] Q. He, A. Mitchell, T. Morcol, S.J.D. Bell, Calcium phosphate nanoparticles induce mucosal immunity and protection against herpes simplex virus type 2, Clin Diagn Lab Immun, 9 (2002) 1021-1024.
[29] T.A. Xia, M. Kovochich, M. Liong, H. Meng, S. Kabehie, S. George, J.I. Zink, A.E. Nel, Polyethyleneimine Coating Enhances the Cellular Uptake of Mesoporous Silica Nanoparticles and Allows Safe Delivery of siRNA and DNA Constructs, Acs Nano, 3 (2009) 3273-3286.
[30] Z.M. Tao, B. Toms, J. Goodisman, T. Asefa, Mesoporous Silica Microparticles Enhance the Cytotoxicity of Anticancer Platinum Drugs, Acs Nano, 4 (2010) 789-794.
[31] J.M. Rosenholm, A. Meinander, E. Peuhu, R. Niemi, J.E. Eriksson, C. Sahlgren, M. Linden, Targeting of Porous Hybrid Silica Nanoparticles to Cancer Cells, Acs Nano, 3 (2009) 197-206.
[32] I.I. Slowing, J.L. Vivero-Escoto, C.W. Wu, V.S.Y. Lin, Mesoporous silica nanoparticles as controlled release drug delivery and gene transfection carriers, Advanced Drug Delivery Reviews, 60 (2008) 1278-1288.
[33] Z.X. Li, J.C. Barnes, A. Bosoy, J.F. Stoddart, J.I. Zink, Mesoporous silica nanoparticles in biomedical applications, Chemical Society Reviews, 41 (2012) 2590-2605.
[34] Y. Wan, D.Y. Zhao, On the controllable soft-templating approach to mesoporous silicates, Chem Rev, 107 (2007) 2821-2860.
[35] P.C. Wilkinson, R.G. White, The role of mycobacteria and silica in the immunological response of the guinea-pig, Immunology, 11 (1966) 229-241.
[36] H. Vallhov, S. Gabrielsson, M. Stromme, A. Scheynius, A.E. Garcia-Bennett, Mesoporous silica particles induce size dependent effects on human dendritic cells, Nano Lett, 7 (2007) 3576-3582.
[37] H. Vallhov, N. Kupferschmidt, S. Gabrielsson, S. Paulie, M. Stromme, A.E. Garcia-Bennett, A. Scheynius, Adjuvant Properties of Mesoporous Silica Particles Tune the Development of Effector T Cells, Small, 8 (2012) 2116-2124.
[38] T. Wang, H. Jiang, Q. Zhao, S. Wang, M. Zou, G. Cheng, Enhanced mucosal and systemic immune responses obtained by porous silica nanoparticles used as an oral vaccine adjuvant: Effect of silica architecture on immunological properties, Int J Pharm, 436 (2012) 351-358.
[39] R.C. Mundargi, V.R. Babu, V. Rangaswamy, P. Patel, T.M. Aminabhavi, Nano/micro technologies for delivering macromolecular therapeutics using poly(D,L-lactide-co-glycolide) and its derivatives, J. Control. Release, 125 (2008) 193-209.
[40] J. Panyam, W.Z. Zhou, S. Prabha, S.K. Sahoo, V. Labhasetwar, Rapid endo-lysosomal escape of poly(DL-lactide-co-glycolide) nanoparticles: implications for drug and gene delivery, FASEB J, 16 (2002) 1217-1226.
[41] L.J. Cruz, P.J. Tacken, R. Fokkink, B. Joosten, M.C. Stuart, F. Albericio, R. Torensma, C.G. Figdor, Targeted PLGA nano- but not microparticles specifically deliver antigen to human dendritic cells via DC-SIGN in vitro, J. Control. Release, 144 (2010) 118-126.
[42] G. Wang, L. Pan, Y. Zhang, Y. Wang, Z. Zhang, J. Lu, P. Zhou, Y. Fang, S. Jiang, Intranasal delivery of cationic PLGA nano/microparticles-loaded FMDV DNA vaccine encoding IL-6 elicited protective immunity against FMDV challenge, PLoS One, 6 (2011) e27605.
[43] S.L. Demento, W. Cui, J.M. Criscione, E. Stern, J. Tulipan, S.M. Kaech, T.M. Fahmy, Role of sustained antigen release from nanoparticle vaccines in shaping the T cell memory phenotype, Biomaterials, 33 (2012) 4957-4964.
[44] C. Coester, P. Nayyar, J. Samuel, In vitro uptake of gelatin nanoparticles by murine dendritic cells and their intracellular localisation, European Journal of Pharmaceutics and Biopharmaceutics, 62 (2006) 306-314.
[45] K. Zwiorek, C. Bourquin, J. Battiany, G. Winter, S. Endres, G. Hartmann, C. Coester, Delivery by cationic gelatin nanoparticles strongly increases the immunostimulatory effects of CpG oligonucleotides, Pharm Res, 25 (2008) 551-562.
[46] J. Klier, S. Fuchs, A. May, U. Schillinger, C. Plank, G. Winter, C. Coester, H. Gehlen, A nebulized gelatin nanoparticle-based CpG formulation is effective in immunotherapy of allergic horses, Pharm Res, 29 (2012) 1650-1657.
[47] M. Hofer, G. Winter, J. Myschik, Recombinant spider silk particles for controlled delivery of protein drugs, Biomaterials, 33 (2012) 1554-1562.
[48] A. Lammel, M. Schwab, M. Hofer, G. Winter, T. Scheibel, Recombinant spider silk particles as drug delivery vehicles, Biomaterials, 32 (2011) 2233-2240.
[49] J. Zhang, E. Pritchard, X. Hu, T. Valentin, B. Panilaitis, F.G. Omenetto, D.L. Kaplan, Stabilization of vaccines and antibiotics in silk and eliminating the cold chain, Proc Natl Acad Sci U S A, 109 (2012) 11981-11986.
[50] A.J. Almeida, E. Souto, Solid lipid nanoparticles as a drug delivery system for peptides and proteins, Adv Drug Deliver Rev, 59 (2007) 478-490.
[51] H. Mishra, D. Mishra, P.K. Mishra, M. Nahar, V. Dubey, N.K. Jain, Evaluation of Solid Lipid Nanoparticles as Carriers for Delivery of Hepatitis B Surface Antigen for Vaccination Using Subcutaneous Route, J Pharm Pharm Sci, 13 (2010) 495-509.
[52] M. Kendall, T. Mitchell, P. Wrighton-Smith, Intradermal ballistic delivery of micro-particles into excised human skin for pharmaceutical applications, J Biomech, 37 (2004) 1733-1741.
[53] H.J. Dean, D. Fuller, J.E. Osorio, Powder and particle-mediated approaches for delivery of DNA and protein vaccines into the epidermis, Comp. Immunol. Microbiol. Infect. Dis., 26 (2003) 373-388.
[54] D. Chen, Y.F. Maa, J.R. Haynes, Needle-free epidermal powder immunization, Expert Review of Vaccines, 1 (2002) 265-276.
[55] H.J. Dean, D.X. Chen, Epidermal powder immunization against influenza, Vaccine, 23 (2004) 681-686.
[56] Y.F. Maa, C. Shu, M. Ameri, C. Zuleger, J. Che, J.E. Osorio, L.G. Payne, D.X. Chen, Optimization of an alum-adsorbed vaccine powder formulation for epidermal powder immunization, Pharm. Res., 20 (2003) 969-977.
[57] C. Rochelle, G. Lee, Dextran or hydroxyethyl starch in spray-freeze-dried Trehalose/Mannitol microparticles intended as ballistic particulate carriers for proteins, J. Pharm. Sci., 96 (2007) 2296-2309.
[58] H. Schiffter, J. Condliffe, S. Vonhoff, Spray-freeze-drying of nanosuspensions: the manufacture of insulin particles for needle-free ballistic powder delivery, J R Soc Interface, 7 (2010) S483-S500.
[59] P.B. Kajjari, L.S. Manjeshwar, T.M. Aminabhavi, Semi-Interpenetrating Polymer Network Hydrogel Blend Microspheres of Gelatin and Hydroxyethyl Cellulose for Controlled Release of Theophylline, Ind Eng Chem Res, 50 (2011) 7833-7840.
[60] J. Ho, Y. Huang, M.K. Danquah, H.T. Wang, G.M. Forde, Synthesis of biodegradable polymer-mesoporous silica composite microspheres for DNA prime-protein boost vaccination, Eur J Pharm Sci, 39 (2010) 412-420.
[61] N.O. Gustavsson, et al., Starch micoparticles, 2004, Patent Number: US 6,692,770 B2
[62] M. Reslow, et al., Microparticles, 2005, Patent Number: US 6,936,278 B2
[63] O.N. Gustafsson, et al., Vaccine composition comprising an immunologically active substance embedded in microparticles consisting of starch with reduced molecular weight, in, 2003, Patent Number: EP1322290 A1
[64] M. Jonsson, et al., Parenterally administrable microparticles, 2007, Patent Number: US 2007/0122484 A1
[65] R.O. Williams III, et al., Process for production of nanoparticles and microparticles by spray freezing into liquid, 2004, Patent Number: US 2004/0022861 A1.
[66] D.N. Osborne, Microparticles for use in needleless injection, 2004, Patent Number: EP1107737 B1.
[67] D.P. Lell, Device for injecting a dust-like or powdery substance into a body tissue, 2008, Patent Number: EP1599242 B1.

## Claims

1. Nano-in-micro particles for intradermal delivery of active substances comprising a matrix of microparticles and nanoparticles carrying at least one active substance, **characterized in that** the nanoparticles are dispersed in the matrix of microparticles.

2. Nano-in-micro particles according to claim 1, wherein the microparticles have a size of 10 to 500 micrometer in at least one dimension.

3. Nano-in-micro particles according to one of the previous claims, wherein the nanoparticles have a size of 1 to 1000 nanometer in at least one dimension.

4. Nano-in-micro particles according to one of the previous claims, wherein the microparticles are selected from group comprising water soluble or not water soluble microparticles, sugar-based microparticles, polysaccharide-based microparticles, dextran-based microparticles or mixtures thereof, microparticles consisting of or comprising a mixture of trehalose, mannitol and dextran (TMD microparticles), sucrose microparticles, cyclodextrin microparticles, hydroxyethyl starch (HES) microparticles, amylase microparticles, pectin microparticles, poly(lactic-co-glycolic acid) (PLGA) microparticles, gelatine-hydroxyethyl cellulose microspheres, gelatine microparticles, PLGA-mesoporous silica microspheres, starch microparticles, amylopectin-based starch microparticles, spider silk microparticles, lipid microparticles, preferably sugars or sugar alcohols, with or without a polysaccharide polymer or other polymers.

5. Nano-in-micro particles according to one of the previous claims, wherein the nanoparticles are selected from the group comprising polymeric nanoparticles, inorganic nanoparticles, hybrid nanoparticles, biodegradable nanoparticles, silica nanoparticles, poly(lactic-co-glycolic acid)(PLGA) nanoparticles, gelatine nanoparticles, silk nanoparticles, solid lipid nanoparticles, pluronic-stabilized polypropylene sulphide (PPS) nanoparticles, ultra-small nanoparticles, poly(lactic acid) (PLA) nanoparticles optionally modified with polyvinyl alcohol (PVA), alginate (ALG) and / or glycolchitosan (GCS), trimethyl chitosan-hyaluronic acid nanoparticles, biodegradable poly(γ-glutamicacid) nanoparticles, gold nanoparticles, calcium phosphate nanoparticles,
mesoporous silica nanoparticles (MSNP), poly(ethylene imine) (PEI) modified hybrid mesoporous silica nanoparticles, PLGA nanoparticles coated with chitosan, gelatine nanoparticles, cationic gelatine nanoparticles, recombinant spider silk nanoparticles, silk fibroin derived from Bombyx mori cocoons-based nanoparticles, lipid-based nanoparticles, solid lipid nanoparticles (SLN).

6. Nano-in-micro particles according to one of the previous claims comprising a densifier.

7. Nano-in-micro particles according to claim 6, wherein the densifier is selected from the group comprising microparticulate densifiers, nanoparticulate densifiers, gold particles, dense salts like calcium carbonate, magnesium carbonate, barium sulphate, hydroxylapatite and its derivatives, magnetite, metal oxides, inert metals, ceramics, hybrid anorganic materials.

8. Nano-in-micro particles according to one of the previous claims comprising at least two different active substances.

9. Nano-in-micro particles according to one of the previous claims, wherein the active substance(s) is / are selected from the group comprising chemical compounds, natural products, extracts of natural products, proteins, peptides, antibodies, insulin, vaccines, subunit vaccines, protein antigens, peptide antigens, live attenuated organisms, inactivated toxins, killed whole organisms, vaccines against influenza, vaccines against cancer, vaccines against viruses, vaccines against human immunodeficiency virus (HIV), vaccines against Hepatitis A, B or C, nucleic acids, DNA, RNA, siRNA, antisense oligonucleotides, steroids, steroid derivatives.

10. Nano-in-micro particles according to one of the previous claims, wherein the active substance is a pharmaceutical compound.

11. Nano-in-micro particles according to one of the previous claims comprising an adjuvant.

12. Composition for intradermal or mucosal delivery comprising nano-in-micro particles as claimed in claims 1 to 11.

13. Pharmaceutical composition for intradermal or mucosal delivery comprising nano-in-micro particles as claimed in claims 1 to 11.

14. Use of nano-in-micro particles, compositions or pharmaceutical compositions according to one of the previous claims for epidermal power injection.

15. Nano-in micro particles, compositions or pharmaceutical compositions according to one of the previous claims for use in intradermal delivery or mucosal delivery of cosmetics, life style products, for use in skin disease treatment, for use in drug administration or immunization, for use in the treatment of skin diseases like e.g. psoriasis, atopic dermatitis, lupus or other rheumatoid skin diseases.
